# EUROPÄISCHE PATENTSCHRIFT

(11) **EP 0 643 015 B1**
(45) Veröffentlichungstag und Bekanntmachung des Hinweises auf die Patenterteilung: **18.09.1996**
(21) Anmeldenummer: 94111686.5
(22) Anmeldetag: 27.07.1994
(51) Int. Cl.: C01B 33/193

(54) **Verfahren zur Herstellung einer Fällungskieselsäure**
Process for the preparation of precipitated silica
Procédé de préparation d'une silice précipitée

(30) Priorität: 07.08.1993 DE 4326576; 05.07.1994 DE 4423493
(43) Veröffentlichungstag der Anmeldung: 15.03.1995
(73) Patentinhaber: Degussa Aktiengesellschaft, D-60311 Frankfurt (DE)
(72) Erfinder: Esch, Heinz, D-53117 Bonn (DE); Kuhlmann, Robert, D-50374 Erftstadt (DE); Neumüller, Matthias, D-63594 Hasselroth (DE); Otto, Karin Dr., D-63450 Hanau (DE); Rausch, Ralf Dr., D-52372 Kreuzau (DE)

(56) Entgegenhaltungen:
- EP-A- 0 062 749
- EP-A- 0 317 378
- EP-A- 0 407 262
- US-A- 3 235 331

## Beschreibung

Diese Erfindung betrifft ein Verfahren zur Herstellung einer Fällungskieselsäure.

Synthetisch hergestellte Kieselsäuren spielen seit vielen Jahren als Bestandteil von Zahnpflegemitteln eine große Rolle. Es ist eine Reihe von Verfahren zur Herstellung von Kieselsäuren, die für die Verwendung in Zahnpasten speziell zugeschnitten sind, bekannt. Diesen Verfahren haften verfahrenstechnische Nachteile an, die sowohl ökonomisch als auch ökologisch nicht zufriedenstellend sind.

So werden bei einer Reihe von Verfahren hohe Mengen an Elektrolyten eingesetzt, die anschließend wieder ausgewaschen werden müssen, um zu den erforderlichen Reinheiten der Endprodukte zu gelangen. Hierdurch entsteht eine erhebliche Salzfracht in den Abwässern (DE-AS 24 46 038).

Bei anderen Verfahren werden zusätzliche Teilschritte für die Bereitung besonderer Fällungsvorlagen eingeschaltet oder zusätzliche hydrothermale Reaktionsschritte mit mehreren Isolationsstufen eingebaut (EP-B 0 317 378).

Aus dem Dokument DE-B 14 67 019 ist ein Verfahren zur Herstellung der Verstärkerfällungskieselsäure mit einer Oberfläche von oberhalb 200 m²/g bekannt. Diese Fällungskieselsäure ist für den Einsatz in Zahnpasten nicht geeignet.

Ein weiterer Nachteil von bekannten Verfahren ist die niedrige Raum-Zeit-Ausbeute bei der Fällung von Kieselsäure durch die notwendige Einführung eines . Unterbrechungsintervalls (Alterungsschritt) oder durch den Einsatz von verdünnten Reaktionskomponenten. Man erhält dadurch üblicherweise nur Feststoffgehalte am Ende der Fällung von ca. 40 - 60 g SiO₂/l (EP-B 0 317 378).

Es wurde nun überraschenderweise gefunden, daß es möglich ist, durch ein einstufiges Fällverfahren Fällungskieselsäuren bei gleichzeitig hoher Raum-Zeit-Ausbeute und ohne den Zusatz von Elektrolyten herzustellen.

Gegenstand der Erfindung ist ein Verfahren zur Herstellung einer Fällungskieselsäure, die eine BET-Oberfläche von 10 - 130 m²/g, eine CTAB-Oberfläche von 10 - 70 m²/g, einen mittleren Teilchendurchmesser von 5 - 20 µm, einen Cu-Abrieb in 10 %iger Glyzerindispersion von 4 - 50 mg und ein Verdickungsverhalten in CMC-Lösung (20 %ige Dispersion) von 300 - 3500 mPa·s aufweist, welches dadurch gekennzeichnet ist, daß man zu einer Wasservorlage, die durch Wasserglaszugabe auf einen pH-Wert von 7,0 bis 9,9 bzw. 10,0 bis 10,7 eingestellt ist, Alkalisilikat (Gewichtsmodul SiO₂ : Alkalioxid = 2,5 - 3,9 : 1) und eine Mineralsäure gleichzeitig hinzugibt, dabei den pH-Wert zwischen 7,0 und 9,9 bzw. 10,0 bis 10,7 konstant hält, wobei die anfängliche Fälltemperatur 50 - 90 °C beträgt und ein Viskositätsanstieg nach spätestens 25 % der Fälldauer eintritt, nach Ereichen eines Kieselsäureghaltes am Ende der Fällung von größer 120 g/l bei einem Fällungs-pH-Wert von 7,0 bis 9,9 bzw. größer 150 g/l, bevorzugt ≧ 160 g/l bis 240 g/l, bei einem Fällungs-pH-Wert von 10,0 bis 10,7 auf pH-Werte ≦ 6, vorzugsweise 3,5, einstellt, den Feststoff durch Filtration abtrennt, wäscht, trocknet und vermahlt.

Zur Filtration kann man Kammerpressen, Bandfilter oder Membranfilterpressen einsetzen.

Zur Trocknung kann man einen Umlufttrockner, Büttnertrockner, Stromtrockner oder ähnliche Trockner einsetzen.

Den verflüssigten Filterkuchen kann man in einem Sprühtrockner trocknen.

Den Filterkuchen kann man ohne Verflüssigung einer Mahltrocknung unterziehen.

Das erfindungsgemäße Verfahren hat den Vorteil, daß bei hohen Feststoffgehalten in der Fällungssuspension, geringem Wasserrückhaltevermögen des Filterkuchen, und folglich dem Einsatz geringer Trocknungsenergien, bei hoher Wirtschaftlichkeit eine breite Palette von Abrasivitäten in der Fällungskieselsäure (und damit bei Verwendung in Zahnpasten in der fertigen Paste) eingestellt werden kann. Dabei kann der Grad der Abrasivität gezielt durch Variation des Feststoffgehaltes in der Fällungssuspension eingestellt werden, wobei begleitende Fällparameterveränderungen, wie zum Beispiel Temperatur, pH-Wert oder Zuführgeschwindigkeit der Reaktanten, weitere Variationen auch in Hinblick auf das Verdickungsverhalten, den Brechungsindex usw. erlauben. Trotz der Variation in den Feststoffgehalten bewegen sich diese bei dem vorliegenden Fällverfahren grundsätzlich auf einem sehr hohen Niveau (≧ 120 g/l, bevorzugt ≧ 160 g/l bis 240 g/l).

Die nach dem erfindungsgemäßen Verfahren hergestellte Fällungskieselsäure kann als Abrasiv- oder Verdickungskomponente in Zahnpastaformulierungen eingesetzt werden. Eine andere Anwendungsmöglichkeit ist der Einsatz als Polier- oder Schleifmittel. Bevorzugt kann die erfindungsgemäß hergestellte Fällungskieselsäure als Abrasivkieselsäure in Zahnpasten eingesetzt werden.

### Beispiele

Die physikalisch-chemischen und anwendungstechnischen Daten der erhaltenen Fällungskieselsäuren sind in Tabelle 1 aufgeführt.

### Beispiel 1

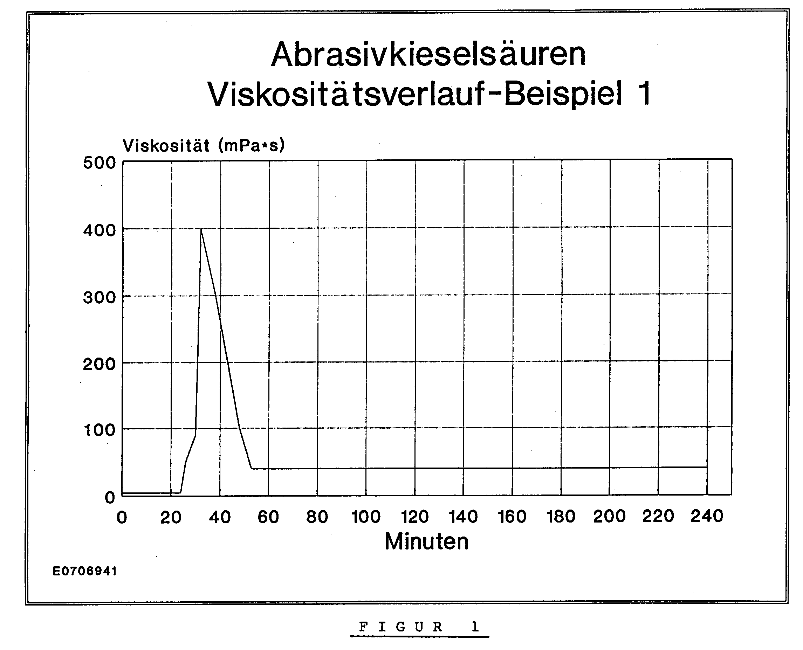

In einem 30-l-Fällgefäß mit indirekter Beheizung werden unter Rühren 12,8 l Wasser vorgelegt und auf 85 °C aufgeheizt. Unter Aufrechterhaltung dieser Temperatur wird zunächst durch Zugabe von wenig Wasserglaslösung (Gewichtsmodul 3,4 : 1 = 26,8 % SiO₂ und 7,85 % Na₂O; Dichte 1,352 g/ml) pH 8,5 eingestellt. Danach wird durch gleichzeitige Zugabe von 56,5 ml/min Wasserglas (Zusammensetzung wie zuvor angegeben) und soviel Schwefelsäure (50 %ig), daß pH 8,5 konstant eingehalten wird, 240 min lang gefällt. Diese Suspension wird anschließend mit Schwefelsäure (50 %ig) bis pH 3,5 gesäuert. Der Kieselsäuregehalt der Suspension beträgt 171 g/l. Der Viskositätsverlauf während der Fällung ist in Figur 1 dargestellt.

Die erhaltene Kieselsäure wird mittels einer Nutsche aus der Suspension abgetrennt, der Filterkuchen mit Wasser gewaschen, bei 105 - 110 °C getrocknet und auf einer Labor-Stiftmühle vermahlen.

### Beispiel 2

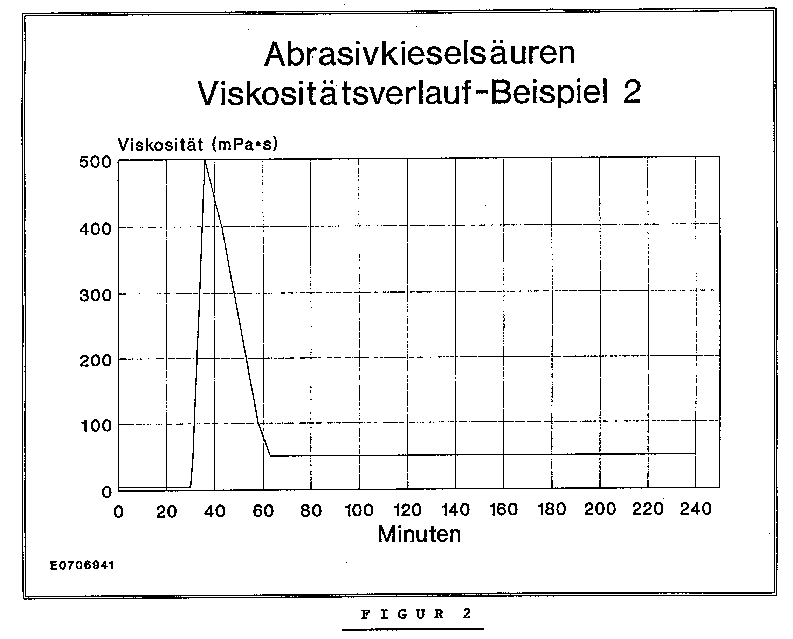

Es wird gemäß Beispiel 1 verfahren mit dem Unterschied, daß der pH-Wert des vorgelegten Wassers mittels Wasserglaslösung auf pH 10,7 eingestellt und dieser pH-Wert während der gleichzeitigen Zugabe von Wasserglas und Schwefelsäure aufrechterhalten wird. Der Kieselsäuregehalt der Suspension beträgt 173 g/l. Der Viskositätsverlauf während der Fällung ist in Figur 2 dargestellt.

### Beispiel 3

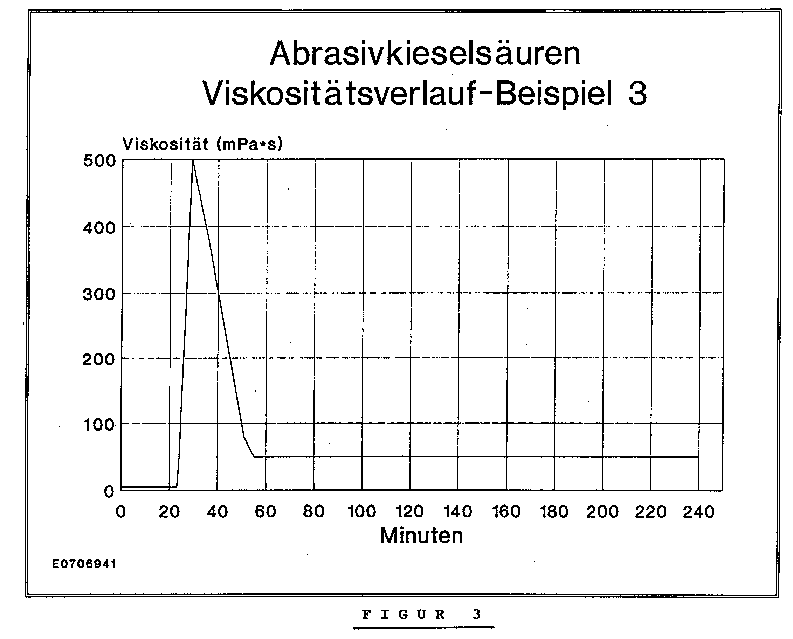

Es wird gemäß Beispiel 2 verfahren. Lediglich der Kieselsäuregehalt in der Suspension wird durch erhöhte Wasserglas- und Schwefelsäuremengen auf 210 g/l erhöht. Der Viskositätsverlauf während der Fällung ist in Figur 3 dargestellt.

### Beispiel 4

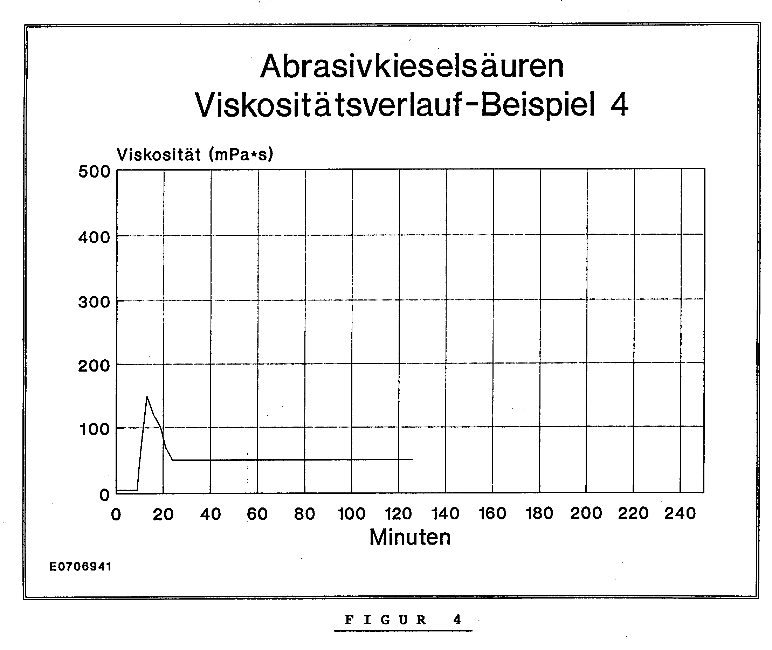

Es wird gemäß Beispiel 1 verfahren mit dem Unterschied, daß durch eine Zugaberate von 56,5 ml/min an Wasserglas und soviel Schwefelsäure (50 %ig) der pH 7 konstant gehalten wird. Die Fälldauer beträgt 126 min. Nach anschließender Sauerstellung auf pH 3,5 wird ein Kieselsäuregehalt von 125 g/l ermittelt. Der Viskositätsverlauf während der Fällung ist in Figur 4 dargestellt.

### Beispiel 5

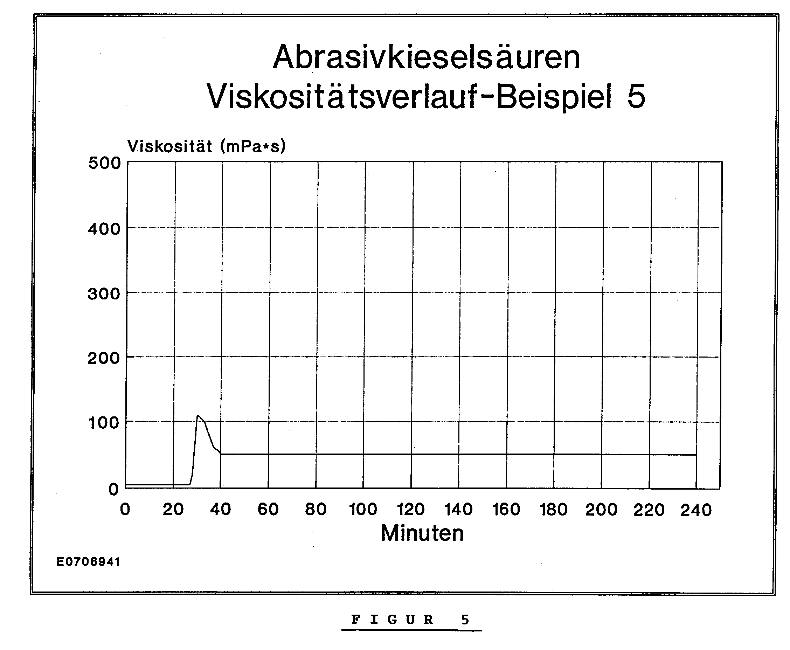

Es wird gemäß Beispiel 1 verfahren, wobei ein Wasserglas mit Gewichtsmodul 2,5 (20,44 % SiO₂ und 8,18 % Na₂O, d = 1,3012 g/ml) eingesetzt und durch Anhebung der Wasserglasdosierung auf 107,3 ml/min und H₂SO₄-Dosierung auf 26,33 ml/min ein Kieselsäuregehalt von 160 g/l in der Suspension eingestellt wird. Der Viskositätsverlauf während der Fällung ist in Figur 5 dargestellt.

**Tabelle 1**

| Beispiel | | 1 | 2 | 3 | 4 | 5 |
|---|---|---|---|---|---|---|
| CTAB-Oberfläche | m²/g | 32 | 45 | 22 | 55 | 25 |
| BET-Oberfläche | m²/g | 37 | 130 | 115 | 65 | 32 |
| Mittlere Teilchengröße C.C. | µm | 12,8 | 11,7 | 13,8 | 12,5 | 14,1 |
| Cu-Abrieb | mg | 17 | 9 | 42 | 16 | 50 |
| CMC-Verdickung | mPa s | 900 | 2100 | 440 | 3200 | 420 |
| Feuchte 2 h 105 °C | % | 3,5 | 3,8 | 4,0 | 3,2 | 3,9 |
| Leitfähigkeit | µS/cm | 70 | 40 | 80 | 110 | 100 |
| Glühverlust | % | 3,0 | 4,6 | 4,7 | 3,2 | 2,8 |
| Gesamtfälldauer G | min | 240 | 240 | 240 | 126 | 240 |
| Viskositätsanstiegspunkt V | min | 24 | 30 | 23 | 9 | 27 |
| Prozentualer Anteil von V an G | % | 10 | 12,5 | 9,6 | 7 | 11,3 |

Die dabei angewandten Methoden sind folgende:
- Die Bestimmung der spezifischen Stickstoff-Oberfläche (BET) erfolgt nach Brunauer - Emmet - Teller mit Hilfe der AREA-meter-Apparatur der Fa. Ströhlein. Die Bestimmung erfolgt gemäß DIN ISO 5794/1 Annex D. Die Originalmethode wurde erstmals in "Journal of the America Chemical Society", 60 (1938) Seite 309 beschrieben. Die Ausheiztemperatur beträgt 160 °C für 1 Stunde.
- Die CTAB-Oberfläche wird durch Adsorption von Cetyltrimethylammoniumbromid bei pH 9 bestimmt (s. Jay, Janzen und Kraus in "Rubber Chemistry and Technology" 44 (1971) 1287).
- Die Teilchenverteilung wird mittels Coulter Counter, Modell TA II der Fa. Coulter Electronics, ermittelt. Zum Einsatz kommt die 100 µm-Kapillare.
- Die Bestimmung der Abrasivität erfolgt nach der Cu-Abriebsmetehode in 10 %iger Glyzerindispersion (157 g Glyzerin wasserfrei, in dem 17 g Kieselsäure 12 min bei 1.500 UpM mit dem Flügelrührer dispergiert werden). Die Abriebmessung erfolgt durch 50.000 Doppelhübe mittels Nylonbürsten an Cu-Blechen (Elektrolyt-Kupfer) in obiger Dispersion. Aus der Differenzwägung erhält man die mg Cu-Abrieb. Literatur: Pfrengle, Fette, Seifen, Anstrichmittel 63 (1961) 445-451 und Reng, Dany, Parfümerie und Kosmetik 59 (1978) 37-45.
- Die Bestimmung des Verdickungsverhaltens erfolgt 20 %ig in einer Carboxymethylcellulose-Lösung (50 g PEG 400, 1 kg 87 %iges Glyzerin, 25 g AKU CMC LZ 855, 925 g Wasser). Die mindestens 1 Tag aber höchstens 2 Wochen alte Testlösung wird mit Kieselsäure versetzt, dispergiert und die Viskosität bestimmt (Brookfield RVT, Spindel 5, 10 UpM, Wert nach 1 min). Die Messung der bei 25 °C thermostatisierten Mischung erfolgt sofort, nach 0,5 h und nach 24 h. Letzterer ist der eigentliche Meßwert.
- Daneben werden die Feuchte (2 h, 105 °C, DIN ISO 787 Teil 2), die Leifähigkeit (4 %ig), der Glühverlust (2 h bei 100 °C, analog DIN 55 921) bestimmt.

## Patentansprüche

1. Verfahren zur Herstellung einer Fällungskieselsäure, die eine BET-Oberfläche von 10 - 130 m²/g aufweist, eine CTAB-Oberfläche von 10 - 70 m²/g, einen mittleren Teilchendurchmesser von 5 - 20 µm, einen Cu-Abrieb in 10 %iger Glyzerindispersion von 4 - 50 mg und ein Verdickungsverhalten in CMC-Lösung (20 %ige Dispersion) von 300 - 3500 mPa·s aufweist, dadurch gekennzeichnet, daß man zu einer Wasservorlage, die durch Wasserglaszugabe auf einen pH-Wert von 7,0 bis 9,9 bzw. 10,0 bis 10,7 eingestellt ist, Alkalisilikat (Gewichtsmodul SiO₂ : Alkalioxid = 2,5 - 3,9 : 1) und eine Mineralsäure gleichzeitig hinzugibt, dabei den pH-Wert zwischen 7,0 und 9,9 bzw. 10,0 bis 10,7 konstant hält, wobei die anfängliche Fälltemperatur 50 - 90 °C beträgt und ein Viskositätsanstieg nach spätestens 25 % der Fälldauer eintritt, nach Erreichen eines Kieselsäuregehaltes am Ende der Fällung von größer 120 g/l bei einem Fällungs-pH-Wert von 7,0 bis 9,9 bzw. größer 150 g/l, bevorzugt ≧ 160 g/l bis 240 g/l, bei einem Fällungs-pH-Wert von 10,0 bis 10,7 auf pH-Werte ≦ 6, vorzugsweise 3,5, einstellt, den Feststoff durch Filtration abtrennt, wäscht, trocknet und vermahlt.

2. Verfahren gemäß Anspruch 2, dadurch gekennzeichnet, daß man zur Filtration Kammerpressen oder Membranfilterpressen einsetzt.

3. Verfahren gemäß Anspruch 2, dadurch gekennzeichnet, daß man zur Trocknung einen Umlufttrockner, Büttnertrockner Stromtrockner oder ähnliche Vorrichtungen, einsetzt.

4. Verfahren gemäß Anspruch 2, dadurch gekennzeichnet, daß man verflüssigten Filterkuchen in einem Sprühtrockner trocknet.

5. Verfahren gemäß Anspruch 2, dadurch gekennzeichnet, daß man den Filterkuchen einer Mahltrocknung unterzieht.

## Claims

1. Process for the production of a precipitated silica which has a BET surface area of 10-130 m²/g, a CTAB surface area of 10-70 m²/g, an average particle diameter of 5-20 µm, a Cu abrasion value in a 10% glycerol dispersion of 4-50 mg and thickening behaviour in a CMC solution (20% dispersion) of 300-3500 mPa·s, which process is characterised in that alkali silicate (weight modulus SiO₂:alkali oxide = 2.5-3.9:1) and a mineral acid are simultaneously added to an initial amount of water which has been adjusted to a pH value of 7.0 to 9.9 or 10.0 to 10.7 by the addition of water glass, the pH value is held constant between 7.0 and 9.9 or 10.0 to 10.7 during addition, wherein the initial precipitation temperature is 50-90°C and an increase in viscosity occurs after at most 25% of the duration of precipitation, the pH value is adjusted to ≦ 6, preferably 3.5, once a silica content of greater than 120 g/l at a precipitation pH value of 7.0 to 9.9 or greater than 150 g/l, preferably ≧ 160 g/l to 240 g/l at a precipitation pH value of 10.0 to 10.7 has been reached at the end of precipitation, the solid is separated by filtration, washed, dried and ground.

2. Process according to claim 2, characterised in that chamber presses or membrane filter presses are used for filtration.

3. Process according to claim 2, characterised in that a circulating air dryer, Büttner dryer, flow dryer or similar device is used for drying.

4. Process according to claim 2, characterised in that fluidised filter cake is dried in a spray drier.

5. Process according to claim 2, characterised in that the filter cake is subjected to mill drying.

## Revendications

1. Procédé pour la fabrication d'une silice précipitée présentant une surface BET de 10 à 130 m²/g, une surface CTAB de 10 à 70 m²/g, un diamètre moyen des particules de 5 à 20 µm, une abrasion de Cu dans une dispersion de glycérine à 10 % de 4 à 50 mg et un comportement d'épaississement dans une solution de CMC (dispersion à 20 %) de 300 à 3500 mPa.s, qui est caractérisé en ce qu'on ajoute, à une charge d'eau disposée au préalable dont le pH est réglé entre 7,0 et 9,9 ou entre 10,0 et 10,7 par une addition de verre soluble, simultanément du silicate alcalin (module pondéral SiO₂:oxyde alcalin = 2,5 - 3,9:1) et un acide minéral, en maintenant le pH constant entre 7,0 et 9,9 ou entre 10,0 et 10,7, la température de précipitation de départ étant de 50 à 90 °C et l'augmentation de la viscosité démarrant au plus tard après 25 % de la durée de précipitation, en ce qu'on règle, après avoir atteint une teneur en silice à la fin de la précipitation supérieure à 120 g/l à un pH de précipitation de 7,0 à 9,9 ou supérieure à 150 g/l, de préférence ≧ 160 g/l à 240 g/l, à un pH de précipitation de 10,0 à 10,7, le pH à une valeur ≦ 6, de préférence à 3,5, en ce qu'on sépare les matières solides par filtration, en ce qu'on les lave, les sèche et le broie.

2. Procédé selon la revendication 2, caractérisé en ce qu'on utilise pour la filtration des presses à plateaux ou des filtres-presses à membranes.

3. Procédé selon la revendication 2, caractérisé en ce qu'on utilise pour le séchage un sécheur à circulation d'air, un sécheur Büttner, un sécheur pneumatique ou des dispositifs analogues.

4. Procédé selon la revendication 2, caractérisé en ce qu'on sèche le gâteau de filtre fluidifié dans un sécheur par pulvérisation.

5. Procédé selon la revendication 2, caractérisé en ce qu'on soumet le gâteau de filtre à un séchage par broyage.
